# EUROPEAN PATENT SPECIFICATION

(11) **EP 0 781 779 B1**
(45) Date of publication and mention of the grant of the patent: **01.03.2006**
(21) Application number: 96120888.1
(22) Date of filing: 27.12.1996
(51) Int. Cl.: C07K 14/78, C12N 9/72, C07K 14/56, A61K 38/21, A61K 47/42

(54) **Nonantigenic stabilizer and physiologically active substance**
Nichtantigenische Stabilisator und physiologisches aktives Mittel
Stabilisateur non-antigénique et substance physiologiquement active

(30) Priority: 27.12.1995 JP 35291895
(43) Date of publication of application: 02.07.1997
(73) Proprietor: Jellice Co., Ltd., Sendai-shi, Miyagi 984-0824 (JP)
(72) Inventor: Sakai, Yasuo c/o Miyagi Kagaku K.K., Sendai-shi Miyagi 982 (JP); Kutsuzawa, Rumiko c/o Miyagi Kagaku k.k., Sendai-shi Miyagi 982 (JP); Onuma, Masamichi c/o Miyagi Kagaku k.k., Sendai-shi Miyagi 982 (JP)
(74) Representative: Sternagel, Fleischer, Godemeyer & Partner

(56) References cited:
- EP-A- 0 230 265
- WO-A-96/41817
- DE-A- 4 244 418
- DATABASE WPI Section Ch, Week 9412 Derwent Publications Ltd., London, GB; Class B04, AN 94-097029 XP002091766 & JP 06 046875 A (AGENCY OF IND SCI & TECHNOLOGY), 22 February 1994
- LI, M. H., ET AL.: "Two-dimensional NMR assignments and conformation of (pro-hyp-gly)10 and a designed collagen triple-helical peptide" BIOCHEMISTRY, vol. 32, no. 29, 27 July 1993, pages 7377-7387, XP002091765
- DATABASE WPI Section Ch, Week 8049 Derwent Publications Ltd., London, GB; Class B04, AN 80-87207C XP002091767 & JP 55 135596 A (NIPPI KK) , 22 October 1980

## Description

### BACKGROUND OF THE INVENTION

### Field of the invention

The invention relates to a nonantigenic stabilizer inducing no anaphylaxis reactions wherein the nonantigenic stabilizer is obtained by specifically decomposing gelatin or collagen using a collagenase, and to a physiologically active substance stabilized thereby.

### Description of Prior Art

Pharmaceutical preparations of various physiologically active substances, particularly proteins, enzymes and vaccine preparations, have been developed for treating and preventing various diseases. A gelatin or collagen (see Japanese Patent Application Laid-0pen No. 54-140715; Japanese Patent Application Laid-Open No. 1-279843; Japanese Patent Application Laid-Open No. 6-234659; Japanese Patent Application Laid-Open No. 51-16488; Japanese Patent Application Laid-Open No. 60-260523; Japanese Patent Application Laid-Open No. 62-149628; and Japanese Patent Application Laid-Open No. 2-49734) or their decomposition products by acid or heat treatment (see Japanese Patent Application Laid-Open No. 49-109520; Japanese Patent Application Laid-Open No. 54-140715; Japanese Patent Application Laid-Open No. 57-114527; Japanese Patent Application Laid-Open No. 63-307827; Japanese Patent Application Laid-Open No. 6-234659; and Japanese Patent Application Laid-Open No. 54-143197) have been used as stabilizer for the preparations. They are used single or in combination with other common stabilizers. A gelatin, collagen and their decomposition products have become popular as stabilizer for various physiologically active substances based upon the experiences that they seldom cause allergic reactions.

On the other hand, a rapid increase of the patients of allergic diseases, referred to as civilizational diseases, has been ever lasting in recent years in Japan as well as countries in Europe and the USA. It is even said that one out of three has some allergic disease now. With such increase of allergic patients as a background, those patients who suffer adverse drug reactions such as anaphylaxis against various physiologically active substances containing a gelatin or collagen as stabilizer which was thought to have little antigenicity/allergenicity (the patients who have gelatin-specific IgE antibody) have recently increased little by little, therefore beginning to make a social problem. In fact, it was not before 1990s that academic reports on these reactions have been seen at times (see Kelso, J. M. et al., Allergy Clin. Immunol., vol. 91. 867-872 and Sakaguchi, M. et al., Infection, Inflammation and Immunity, vol. 26, 48-50). It is an important problem since adverse drug reactions such as anaphylaxis should not be caused by a pharmaceutical preparation of a physiologically active substance originally used for treating and preventing various diseases.

The inventors therefore intensively and repeatedly studied on those derivatives of gelatin and collagen that show no antigenicity or allergenicity in view of such actuality. As a result, we found that a peptide composite with a molecular weight ranging up to 1,000 which has no antigenicity, maintaining (Gly-X-Y)ₙ, and a specific amino acid sequence for collagen, by making a single collagenase or the enzyme immobilized on various carriers directly act on materials containing gelatin or collagen to perform a specific enzymolysis (see Japanese Patent Application Laid-Open No. 7-82299).

However, no use of the peptide composite shown by the inventors in Japanese Patent Application Laid-Open No. 7-82299 as stabilizer could be thought since the gelatin whose molecular weight of not more than about 10,000 was conventionally thought to have little stabilizing effects of urokinase as shown in Japanese Patent Application Laid-Open No. 54-80406. The peptide composite shown in Japanese Patent Application Laid-Open No. 7-82299 also had a problem of limitation in raising the yield due to the narrow range of molecular weight.

The invention is made to solve such conventional problems and its object is to provide a nonantigenic stabilizer obtainable with a high yield which induces no anaphylaxis and has an effect of stabilizing physiologically active substances, and to present physiologically active substances stabilized thereby.

### BRIEF SUMMARY OF THE INVENTION

The inventors intensively and repeatedly studied derivatives of gelatin and collagen that show no antigenicity or allergenicity, and consequently found that those peptide composites with an amino acid sequence (Gly-X-Y)ₙ that is obtained by a specific decomposition of gelatin or collagen using a collagenase and has a molecular weight not more than 20,000 not only show no antigenicity/allergenicity but also have an action to stabilize various physiologically active substances.

The present invention thus relates to the use as a nonantigenic stabilizer of a peptide whose molecular weight is not more than 20.000 and whose amino acid sequence is (Gly-X-Y)ₙ that is obtained by a specific decomposition of gelatin or collagen using a collagenase. Particularly, the nonantigenic stabilizer involved in the present invention preferably comprises the peptide composite which is obtained by a specific decomposition of gelatin or collagen using a collagenase, and contains not less than 70% of peptide whose molecular weight is not more than 20.000 and whose amino acid sequence is (Gly-X-Y)ₙ. In particular, the nonantigenic stabilizer involved in the present invention preferably contains at least 85%, more preferably 95% of said peptide to increase nonantigenicity.

The physiologically active substance involved in the present invention is, for example, a virus, vaccine, one of various cytokines or antibiotics. The "physiologically active substance" herein refers to a concept including a virus, vaccine, various cytokines, or antibiotics as well as components such as proteins, enzymes, bacteria, hormones, nucleic acids, antibody, or those pharmaceutical preparations for the purpose of treatment or prevention that have these substances as active ingredients. The physiologically active substance involved in the present invention may also be those antibodies or enzymes that are prepared by genetic engineering techniques.

More concrete examples for the physiologically active substance include hormones/proteins/cytokines such as insulin, kallikrein, aprotinin, prolactin, chorionic gonadotrophin, luteinizing hormone, transforming factors (TGFα, TGFβ and TGFγ etc.), platelet-derived growth factor (PDGF), fibroblast growth factor (FGF), tissue plasminogen activator (t-PA), other stimulators and cytokines; enzymes such as urokinase, cytochrome C, ribonuclease, papain, chymotrypsin, pepsin and trypsin; clotting factors such as plasmin, thrombin and antithrombin; nucleic acid related substances such as adenosine triphosphate (ATP), nucleotides and nucleic acids; antibiotics such as cephem, penicillin, tetracycline, chloramphenicol and polypeptide antibiotics; and other biological components such as prostaglandin.

Since the physiologically active substances are generally unstable, often changed during storage with the passing of time and their activity is greatly reduced, components derived from gelatin or collagen are often added as stabilizer in a practical use as described above. The use of the nonantigenic stabilizer involved in the present invention for the physiologically active substances as stabilizer allows preparing those pharmaceutical preparations of physiologically active substances, etc. that will not cause adverse drug reactions such as anaphylaxis.

There is a fear of antigenicity appearing with even those peptides with an amino acid sequence (Gly-X-Y)ₙ that are obtained by specific decomposition of gelatin or collagenase using a collagenase if they have a molecular weight over 20,000. The nonantigenic stabilizer involved in the present invention has a molecular weight which is not more than 20,000. Thus it can be prepared with a higher yield from the same raw material than that with a molecular weight not more than 1.000. The nonantigenic stabilizer involved in the present invention preferably has a molecular weight not more than 10,000 to raise its nonantigenicity. Preferably, the percentage of the peptides whose molecular weight is not over 10,000 in those peptides of the nonantigenic stabilizer involved in the present invention whose molecular weight is not more than 20,000 and whose amino acid sequence is (Gly-X-Y)ₙ is not less than 90%.

In the formula (Gly-X-Y)ₙ of the amino acid sequence. X and Y are any amino acid residues other than Gly, e.g. Pro and Hyp, and n is a natural number.

The physiologically active substance involved in the present invention is characterized by containing 0.005-15 percent by weight of the nonantigenic stabilizer involved in the present invention. The physiologically active substance involved in the present invention preferably contains 0.01-10 percent by weight of the nonantigenic stabilizer. However, the amount of the physiologically active substance to add to the nonantigenic stabilizer cannot uniformly be defined since it varies with the kind of the physiologically active substance to add to and the type of the pharmaceutical preparation.

The peptide as the nonantigenic stabilizer may be used alone or may be used in combination with other commonly used stabilizers Examples for the combined other general stabilizers include sorbitol, inositol, mannitol, saccharose, lactose, monosodium glutamate, dextran, glycerol and amino acids.

For starting materials of the nonantigenic stabilizer involved in the present invention, a gelatin or collagen, particularly the collagen or gelatin prepared from fresh bones, skin, tendon or cartilage derived from animals including a bovine and pig as raw materials may be used. On this occasion, the degree of purification of the collagen or gelatin is preferably higher, but no degree of the purification is specifically required if the purity or specificity of the collagenase used is excellent or a process for purification can be incorporated after an enzyme treatment. A more important selection criterion for these starting materials is a relation to the use of the manufactured nonantigenic stabilizer, which the materials are preferably selected upon.

For the collagenase used for enzymolysis when the nonantigenic stabilizer involved in the present invention is Produced, those enzymes derived from bacteria such as Clostridium histolyticum and Streptomyces parvulus actinomycetes or fungi, etc. that specifically cleave the amino acid side of the glycine in the amino acid sequence (Gly-X-Y)ₙ which is characteristic of collagen. The collagenase may also be one of the collagenase having similar substrate specificity that is product of gene recombination obtained by incorporating the enzyme gene into a specific vector with genetic engineering to allow production with other microorganisms such as lactic acid bacteria and yeast or animals.

It is a common way of thinking in case of the peptides with several thousands or more of molecular weight that they may generally maintain the antigenicity of the raw material partially at least. In actual fact, as shown in the undermentioned example, activity of approximately 1/15 to 1/50 of the antigenicity of gelatin remained in case.of the products of decomposition of gelatin which had a molecular weight ranging from 500 to 20,000 that were prepared by treating gelatin or collagen with acids or heating. Further, the antigenicity as gelatin also remained approximately 1/50 to 1/200 in case of those gelatin composites obtained by treatment not with a decomposing method by acids or heating but with enzymolysis by a single or mixture of more than two general protease such as pepsin, trypsin, papain, chymotrypsin, pancreatin or actinase. Thus it is supposed that the attempt for lower molecular weight by a simple decomposition may not be sufficient for eliminating the antigenicity of gelatin, and that there may be a close relation between the elimination of antigenicity and a method for decomposing gelatin.

A critical point for producing the nonantigenic stabilizer involved in the present invention is the purity of the collagenase used. A collagenase, generally prepared from various bacterial bodies, is sometimes contaminated by other proteases. The much contained impure enzymes results in the decomposition of proteins other than gelatin or collagenase in a raw material or the nonspecific decomposition of gelatin or collagen itself by the impure enzymes. In this case, the quality of the nonantigenic stabilizer purified may be reduced, thereby causing induction of anaphylaxis. It is therefore necessary to take enough care for the purity of collagenase used as well as the substrate specificity

The collagenase may be used in a free form or may be used as an immobilized enzyme in which the collagenase is combined with various carriers by physical adsorption or chemical bonding. As a method for enzymolysis by the collagenase, (a) a batch process, (b) a column process, or (c) a method combining the two may be used. The manufacturing line by the methods (a)-(c) and the form of collagenase used may be freely combined. The manufacture of the nonantigenic stabilizer involved in the present invention may be performed in accordance with the method described in Japanese Patent Application Laid-Open No. 7-82299, and also with other methods. It is rather preferable to select a respective method for gelatin or collagen as raw material or to select a method suitable for maintaining the specificity or purity of collagenase.

More concretely, as an example for the method for manufacturing the nonantigenic stabilizer involved in the present invention, the nonantigenic stabilizer may be manufactured as shown in the example below from gelatin or collagen as starting material by a bioreactor system with a batch or column process using an immobilized enzyme for better enzyme yield. Specifically, a collagenase may be combined with various carriers, e.g. CHITOPEARL, by physical adsorption or chemical bonding, the complex may be packed in a column for chromatography, and a solution of gelatin solubilized or a collagen denatured at such a temperature that no decomposition occurs, preferably 40-45°C may be allowed to pass through the column for enzymolysis. The speed of transferring the raw material collagen may be properly selected in accordance with the activity of the immobilized enzyme and the needed degree of decomposition.

While the following example serves to specifically illustrate the present invention, it is not intended to limit any scope of the invention. The use of the peptide as nonantigenic stabilizer of the present invention for physiologically active substances can be performed in accordance with the methods for using already known stabilizers for physiologically active substances.

### Example

After dissolving 50 g of highly purified gelatin (produced by Jellice Co., Ltd) in 1.000 mL of 20 mM Tris-HCL buffer solution (pH 7.4)/0.1 M NaCl by heating, the solution was cooled to 50°C. An immobilized enzyme was prepared by combining 100 mg of collagenase (produced by Washington. Ltd.; a highly purified product from type IV) with 50 g of CHITOPEARL (Fuji Boseki Co., Ltd.) using two crosslinking reagents. The absorbances at 280 nm were measured before and after the binding so as to calculate a percentage of the collagenase bound to the carrier. The percentage was not less than 99%. When the immobilized enzyme was used, it was packed in a tandem column bioreactor with a pH sensor placed between the columns and washed and equilibrated with 20 mM Tris-HCl buffer solution (pH 7.4)/0.1 M NaCl. The system for measuring pH comprises a pH sensor placed between the columns of the tandem columns which senses a change of pH and a tube connected to it from which a concentrated Tris buffer solution flows into it.

The highly purified collagen prepared in the above process was applied to the collagenase immobilized vertical tandem columns to perform enzymolysis by a column method. The flow rate was kept at 50-80 mL/minute and the column temperature was maintained at 39 ± 1 °C at the while. The liquid after the end of enzyme reaction which flew out from the last (second) column was fractionated and filtered with a 0.45*µ*m filter.

The filtrate was powdered by a spray dryer, then purified by gel filtration using a gel filtration system (trade name: Superdex G-30; Pharmacia) or by reversed phase chromatography using an ODS column (produced by YMC) to obtain peptide composites respectively with not more than 1,000 or not more than about 20,000 of molecular weight. The peptide composites respectively with not more than 1,000 or not more than about 20,000 of molecular weight is used as the nonantigenic stabilizer in the example of the present invention.

The molecular weight distribution of the respective peptide composites was measured by high performance liquid chromatography: HPLC (LC-10A from Shimazu Seisakusho Ltd.; column: Superdex peptide). The respective samples were filtered by a 0.2 µm membrane filter before injecting to the HPLC column. Table 1 summarizes the mean molecular weight, range of molecular weight and population of peptides with molecular weight of 500 or below for the fraction of peptides with not more than 1,000 of molecular weight and the fraction of peptides with not more than about 20,000 of molecular weight. The results indicate that the manufacture of the peptides with not more than about 20,000 of molecular weight provides a higher yield than that of the peptides with not more than 1,000 of molecular weight.

**[Table 1]**

| Peptide composite | Mean molecular weight (M. W.) | Peptide of ≦1,000 (%) | Peptide of ≦500 (%) |
|---|---|---|---|
| ≦20,000 | 3,200 | 15,2 | 5,6 |
| ≦ 1,000 | 530 | 99,8 | 52,2 |

After freeze-drying the respective peptide composites, i.e. the peptide composite with not more than 1,000 of molecular weight and the peptide composite with not more than about 20,000 of molecular weight, respective NH2 terminal amino acids and the second amino acid from the NH2 terminus were tested by the Edman decomposition method. As a result, it was revealed that respectively 95.7% and 97.5% of the amino acids on the NH2 side of the both were glycine, demonstrating that they have a (Gly-X-T)ₙ structure which is characteristic of the nonantigenic stabilizer of the present invention. A test method for elimination of the antigenicity/allergenicity of the peptide composites obtained in the example and the results are shown below.

### [Preparation of gelatin antiserum (IgG type)]

Gelatin derived from bovine skin and pig skin was dissolved in PBS to adjust the concentration to 2 mg/mL and the solution was filtered with a 0.22 µm filter. An emulsion was prepared by mixing the same volumes of the filtrate and the Freund's complete adjuvant and injected to three rabbits, 1 mL each. After three weeks, an emulsion was prepared by mixing the same volumes of the solution of same peptide composite and the Freund's complete adjuvant and similarly injected to the rabbits. The procedure was repeated three times, and antisera were obtained on the seventh day after the last immunization.

### [Preparation of gelatin antiserum (lgE type)]

Gelatin derived from bovine skin and pig skin was dissolved in PBS to adjust the concentration to 2 mg/mL and the solution was sterilized by filtration with a 0.22*µ*m filter. Aluminum hydroxide (Alum) was added to the filtrate as precipitant, and the precipitate was washed and dissolved to prepare a 100*µ*g/mL solution, and the solution was subjected to intracutaneous injection to three guinea pigs, 1 mL each. After 4 weeks, additional immunization was similarly performed, and antisera were obtained after further 3-5 days.

### [Preparation of gelatin sensitized immuno ball]

A gelatin sensitized immuno ball in which gelatin derived from bovine skin or pig skin was immobilized on an aminated polystyrene ball (produced by Sumitomo Bakelite Co., Ltd..) activated by two crosslinking agents and blocked by bovine serum albumin or a surface active agent.

### [Test 1]

### [Enzyme immunoassay (antigenicity test-1)]

### (Test for antigenicity by inhibitory reaction)

Respectively 200*µ*m of i) The peptide composite with not more than about 20,000 of molecular weight obtained in the example (the nonantigenic stabilizer of the example), ii) the peptide with not more than 1,000 of molecular weight obtained in the example (the nonantigenic stabilizer of the example), iii) partially decomposed gelatin with molecular weight ranging from 200 to 7,000 obtained by thermolysis (comparative example), iv) enzymolytic gelatin with molecular weight ranging from 500 to 12,000 prepared by enzymolysis with trypsin and pepsin (comparative example) and v) gelatin (comparative example) were added to a gelatin sensitized immuno ball, then 200 µL of either said rabbit gelatin antiserum of lgG type or said guinea pig gelatin antiserum of lgE type was added and the mixture was allowed to react at 37°C for 30 minutes to perform competitive reactions of the respective components of i) to v) in the reaction system of antiserum and gelatin antigen. Then the reaction product was washed and submitted to a secondary reaction with a horseradish root peroxidase (HRP) labeled goat anti-rabbit-lgG antiserum complex (Cosmo Bio) or horseradish root peroxidase (HRP) labeled goat anti-guinea-pig-lgE antiserum complex (Cosmo Bio). After a reaction at 37°C for 1 hour, the remaining activity of the HRP labeled complex bonded to the immuno ball was measured.

The activity of the HRP labeled complex was measured by allowing the complex to react with a "substrate solution" for measuring activity containing 0.2% o-phenylenediamine hydrochloride (OPD) and 0.02% hydrogen peroxide at 37°C for 20 minutes, then the reaction was stopped with a dilute sulfuric acid solution and by measuring the absorbance at 492 nm of wavelength with a spectrophotometer (V-550; JASCO). The antigenicity and allergenicity of respective components in i) to v) were studied from the degree of competitive reactions by measuring the activity of the HRP labeled complex. Table 2 shows the' results. From the results in Table 2. the nonantigenic stabilizer of the example in i) and ii) has 0% of inhibition rate, indicating that they have no antigenicity while those of the comparative examples in iii) to v) have some.

**[Table 2]**

| Sample (treating method) | Molecular weight | Inhibition rate (%) |
|---|---|---|
| ① Stabilizer of example | ≦ 20,000 | 0 |
| ② Stabilizer of example | ≦ 1,000 | 0 |
| ③ Thermolytic gelatin | 200 - 7,000 | 8.1 |
| ④ Trypsin/pepsin enzymolytic gelatin | 500 - 12,000 | 0.6 |
| ⑤ Gelatin | (-) | 100 |

### [Test.2]

### [Passive Cutaneous Anaphylaxis (PCA) (antigenicity test-2)]

Using a sterilized physiological saline, a 1/2 serial dilution (1/10, 1/20, 1/40, 1/80 and 1/160) of guinea pig anti-bovine-gelatin antiserum was prepared and 50*µ*l of respective diluted serum solutions were intracutaneously injected to the back of two SD rats each (male, 8 weeks of age; four rats in total) whose back hair was clipped. After 24 hours, 1.0 mL of a 0.6% Evans blue solution containing 1 mg of the peptide composite with not more than about 20,000 of molecular weight obtained in the example (the nonantigenic stabilizer of the example) was injected to the caudal vein of one of the rats. Similarly, 1.0 mL of a 0.6% Evans blue solution containing 1 mg of bovine gelatin was injected to the caudal vein of the second SD rat as a positive control for the peptide composite in ii). After 60 minutes, all the four rats were sacrificed and the back skin was peeled to observe purpura and measure the size. When the size equaled to, or more than 10 mm, its judgment was (++) or (+++). When the size ranged from 9 mm to 5 mm or 4 mm to 1 mm the judgments were respectively (+) and (±). When no purpura appeared, the judgment was (-). The results are shown in Table 3. From the results in Table 3. no purpura appears even with 1/10 serial dilution of the peptide composite with not more than about 20,000 of molecular weight obtained in i) the example showing that the composite has no antigenicity while ii) bovine gelatin causes purpura even with the 1/160 serial dilution.

**[Table 3]**

| Sample | Antiserum serial dilution | Judgment of results of PCA reaction |
|---|---|---|
| Bovine gelatin | 1/10 | (++ - +++) |
| | 1/20 | (++) |
| | 1/40 | (+) |
| | 1/80 | (+) |
| | 1/160 | (±) |
| Stabilizer of example | 1/10 | (-) |
| | 1/20 | (-) |
| | 1/40 | (-) |
| | 1/80 | (-) |
| | 1/160 | (-) |

### [Test 3]

### [Fluorescent enzyme immunoassay (antigenicity test-3)]

### (Test for allergenicity by inhibitory reaction)

Using sera (containing gelatin specific IgE) collected from six patients showing allergy against gelatin (Patient A to F in Table 4) and the gelatin sensitized immuno ball shown in example 7, the degree of inhibition of the titer of gelatin specific lgE antibody by the nonantigenic stabilizer of the example by measuring the fluorescence of fluorescent substrate decomposed by the labeled enzyme on performing the fluorescent enzyme immunoassay to measure the gelatin specific lgE antibody that of a patient who was found positive by fluorescent enzyme immunoassay. The fluorescence intensity was measured at 495 nm of excitation wavelength and 520 nm of fluorescence wavelength using a fluorophotometer (FP777; JASCO). A β-galactosidase labeled mouse anti-human-lgE antibody was used as the second antibody to detect the gelatin specific lgE antibody, and the enzyme activity was assayed using a fluorescent substrate. The results are shown in Table 4.

**[Table 4]**

| Sample | | Titer of antibody from respective patients of allergy (fluorescence intensity : F.I.) | | | | | |
|---|---|---|---|---|---|---|---|
| Sample name | Molecular weight | A | B | C | D | E | F |
| Control group : no sample | (-) | 8,890 | 1,542 | 9,004 | 6,663 | 1,034 | 8,226 |
| Stabilizer of Example | ≦ 20,000 | 8.778 | 1,608 | 8,905 | 6,560 | 1,029 | 8,007 |
| Stabilizer of Example | ≦ 1,000 | 8,995 | 1,621 | 9,550 | 6,696 | 1,150 | 8,544 |
| Gelatin | (-) | 267 | 59 | 822 | 85 | 41 | 307 |
| Thermolytic gelatin | 200-7,000 7,000 | 594 | 115 | 978 | 1.601 | (-) | (-) |
| Trypsin/pepsin enzymolytic gelatin | 500-12,000 | 1,007 | 105 | 855 | 1,025 | (-) | (-) |

As shown in Table 4, no reduction of the titer (fluorescence intensity) of antibody by inhibitory reaction was observed with the peptide with not more than about 20,000 of molecular weight and the peptide with not more than about 1,000 of molecular weight obtained in the example (the nonantigenic stabilizers of the example) revealing that the peptides have no reactivity with a gelatin specific IgE antibody. On the contrary it was shown that a strong inhibition was caused by the original raw material gelatin thermolytic gelatin or gelatin decomposed by a non-specific protease and they reacts well with a gelatin specific IgE antibody, revealing one of the cause for anaphylaxis. Performing the test by the example may inform you whether respective peptide composites have reactivity with the gelatin specific IgE which induces type 1 allergy or not.

### [Test 4]

### [Stabilization for urokinase preparation]

An urokinase preparation was prepared by adding the peptide with not more than 20,000 of molecular weight obtained in the example (the nonantigenic stabilizer of the example) or mannitol to urokinase and freeze-drying following the recipe below:
[Recipe] Urokinase : 160,000 IU; Peptide obtained in the example (nonantigenic stabilizer of the example) : 0 or 0.8 g; and Mannitol : 0 g or 0.2 g. Dilute with distilled water for injection to 100 mL of total volume.

The stability test for the prepared urokinase preparation was performed by dissolving the freeze-dried urokinase preparation with a physiological saline, allowing the solution to stand at 30°C for 0.4. 24 and 48 hours, then measuring the percentage of residual activity with a urokinase measurement two-step method [see Drug Research 5. 295, 1974, Titration of urokinase]. The results are shown in Table 5. It is known from the results in Table 5 that the stability of a urokinase preparation is more increased by addition of the peptide obtained in the example (nonantigenic stabilizer of the example) than by no addition or addition of mannitol.

**[Table 5]**

| Additive | | Initial urokinase activity (KU) | Percentage of urokinase residual activity (%) | | | |
|---|---|---|---|---|---|---|
| Stabilizer of Example | Mannitol | | 0 hr | 4 hrs | 24 hrs | 48 hrs |
| (+) | (+) | 1,600 | 100 | 100 | 100 | 100 |
| (+) | (-) | 1,600 | 100 | 100 | 100 | 99 |
| (-) | (+) | 1,500 | 100 | 96 | 90 | 82 |
| (-) | (-) | 1,500 | 100 | 95 | 89 | 78 |

### [Test 5]

### [Stabilization of freeze-dry urokinase preparation]

The freeze-dry urokinase preparation prepared in Test 4 was stored at 45°C and the residual urokinase activity after a period (1-3 months) was measured similar to Test 4 to study the stabilization effect of the peptide with not more than 20,000 of molecular weight obtained in the example (nonantigenic stabilizer of the example). The results are shown in Table 6. It is known from the results in Table 6 that the stability of a urokinase preparation is more increased by addition of the peptide with not more than 20,000 of molecular weight obtained in the example (nonantigenic stabilizer of the example) than by no addition or addition of mannitol.

**[Table 6]**

| Additive | | Initial urokinase activity (KU) | Percentage of urokinase residual activity (%) | | | |
|---|---|---|---|---|---|---|
| Stabilizer of Example | Mannitol | | After 0 month | After 1 month | After 2 months | After 3 months |
| (+) | (+) | 1,600 | 100 | 99 | 100 | 100 |
| (+) | (-) | 1,600 | 100 | 100 | 98 | 99 |
| (-) | (+) | 1,500 | 100 | 95 | 89 | 83 |
| (-) | (-) | 1,500 | 100 | 93 | 88 | 80 |

### [Test 6]

### [Stabilization of interferon preparation]

An interferon-α preparation was prepared by adding the peptide with not more than 20,000 of molecular weight obtained in the example and other stabilizers as controls to interferon-α produced by genetic engineering (produced by Cosmo Bio; specific activity: 10⁷ U/mg) then freeze-drying following the recipe below:
[Recipe] lnterferon-α: 10⁶ U; Peptide with not more than 20,000 of molecular weight obtained in the example (nonantigenic stabilizer of the example) : 0% or 0.2%; and Other stabilizers : 0% or 0.2%. Dilute with distilled water for injection to 200 mL of total volume.

The freeze-dry preparations was stored in a refrigerator (5-10°C) for about one month, then they were dissolved with physiological saline and the solution was allowed to stand at 30°C for 30 minutes or 4 hours, then the residual interferon activity was measured. The percentage of residual activity was assayed and the results are shown in Table 7. It is known from the results in Table 7 that the stability of an interferon-α is more increased by addition of the peptide with not more than 20,000 of molecular weight obtained in the example (nonantigenic stabilizer of the example) than by no addition or addition of only other stabilizers except human serum albumin.

**[Table 7]**

| Additive | | Percentage of residual activity (%) | |
|---|---|---|---|
| Stabilizer of Example | Other stabilizers | After 30 minutes | After 4 hrs |
| (+) | (-) | 95 | 87 |
| (-) | Human serum albumin | 97 | 89 |
| (-) | Phosphate buffered saline | 0 | 0 |
| (-) | (-) | 0 | 0 |

### Advantage of the Invention

According to the nonantigenic stabilizer involved in the present invention, no anaphylaxis reactions are induced due to eliminated antigenicity as well as characteristics of amino acid sequence of gelatin or collagen, thereby advantageous for a stabilizer of physiologically active substances for treatment and prevention. According to the nonantigenic stabilizer involved in the present invention, a wider range of molecular weight than that of conventional non-antigenic peptide composites can be provided, and the yield can be increased.

## Claims

1. Use of a peptide, whose molecular weight is not more than 20,000 and whose amino acid sequence is (Gly-X-Y)ₙ, wherein X and Y are any other amino acid residues other than Gly and n is a natural number, and which is obtained by specifically decomposing gelatin or collagen using collagenase, as a nonantigenic stabilizer.

2. The use of claim 1, wherein the nonantigenic stabilizer contains not less than 70 % of a peptide whose molecular weight is not more than 20,000 and whose amino acid sequence is (Gly-X-Y)ₙ.

3. The use of claim 2, wherein the stabilizer contains not less than 85 % of said peptide.

4. A physiologically active substance, which contains 0,005-15 percent by weight of a nonantigenic stabilizer obtained by specifically decomposing gelatin or collagen using collagenase, wherein the nonantigenic stabilizer is mainly composed of a peptide whose molecular weight is not more than 20,000 and whose amino acid sequence is (Gly-X-Y)ₙ, wherein X and Y are any other amino acid residues other than Gly and n is a natural number.

5. The physiologically active substance according to claim 4, which is virus, vaccine, various cytokines or antibiotics.

6. The physiologically active substance according to claim 4, which is an antibody or enzyme prepared by a genetic engineering technique.

## Patentansprüche

1. Verwendung eines Peptides als nicht-antigenisches Stabilisierungsmittel, wobei das Peptid ein Molekulargewicht von nicht mehr als 20.000 aufweist, und dessen Aminosäuresequenz (Gly-X-Y)ₙ ist, wobei X und Y jeder andere Aminosäurerest außer Gly bedeuten und wobei n eine natürliche Zahl darstellt, und wobei das Peptid erhalten wird durch spezifischen Abbau von Gelatine oder Kollagen unter Verwendung von Kollagenase.

2. Die Verwendung nach Anspruch 1, wobei das nicht-antigenische Stabilisierungsmittel nicht weniger als 70% eines Peptides enthält, dessen Molekulargewicht nicht mehr als 20.000 beträgt und dessen Aminosäuresequenz (Gly-X-Y)ₙ ist.

3. Die Verwendung nach Anspruch 2, wobei das Stabilisierungsmittel nicht weniger als 85% des Peptides enthält.

4. Eine physiologisch aktive Substanz, die 0,005 bis 15 Gewichtsprozent eines nicht-antigenischen Stabilisierungsmittels enthält, das erhalten wird durch spezifischen Abbau von Gelatine oder Kollagen unter Verwendung von Kollagenase, wobei das nicht-antigenische Stabilisierungsmittel hauptsächlich aus einem Peptid besteht, dessen Molekulargewicht nicht mehr als 20.000 beträgt und dessen Aminosäuresequenz (Gly-X-Y)ₙ ist, wobei X und Y jeder andere Aminosäurerest außer Gly bedeuten und wobei n eine natürliche Zahl darstellt.

5. Die physiologisch aktive Substanz nach Anspruch 4, **dadurch gekennzeichnet, dass** sie ein Virus, ein Vakzin, verschiedene Cytokine oder Antibiotika darstellt.

6. Die physiologisch aktive Substanz nach Anspruch 4, **dadurch gekennzeichnet, dass** sie einen Antikörper oder ein Enzym darstellt, die durch ein gentechnisches Verfahren hergestellt wurden.

## Revendications

1. Utilisation d'un peptide dont le poids moléculaire ne dépasse pas 20 000 et dont la séquence d'acides aminés est (Gly-X-Y)ₙ, dans laquelle X et Y sont n'importe quels autres résidus d'acides aminés que Gly et n est un entier naturel, et qui est obtenu par décomposition spécifique de gélatine ou collagène en utilisant de la collagénase comme stabilisant non antigénique.

2. Utilisation de la revendication 1, dans laquelle le stabilisant non antigénique contient au minimum 70% d'un peptide dont le poids moléculaire ne dépasse pas 20 000 et dont la séquence d'acides aminés est (Gly-X-Y)ₙ.

3. Utilisation de la revendication 2, dans laquelle le stabilisant contient au minimum 85% dudit peptide.

4. Substance physiologiquement active qui contient de 0,005 à 15% en poids d'un stabilisant non antigénique obtenu par décomposition spécifique de gélatine ou de collagène en utilisant de la collagénase, dans laquelle le stabilisant non antigénique est principalement composé d'un peptide dont le poids moléculaire ne dépasse pas 20 000 et dont la séquence d'acides aminés est (Gly-X-Y)ₙ, dans laquelle X et Y sont n'importe quels autres résidus d'acides aminés que Gly et n est un entier naturel.

5. Substance physiologiquement active selon la revendication 4 qui est un virus, un vaccin, plusieurs cytokines ou des antibiotiques.

6. Substance physiologiquement active selon la revendication 4 qui est un anticorps ou un enzyme préparé par une technique de l'ingénierie génétique.
